# EUROPEAN PATENT APPLICATION

(11) **EP 4 112 636 A1**
(43) Date of publication of application: **04.01.2023**
(21) Application number: 21761114.4
(22) Date of filing: 26.02.2021
(51) Int. Cl.: C07K 14/195, C12N 15/31, C12N 15/63, A61K 39/39, A61K 38/16, A61P 35/00

(54) **TUMOR IMMUNE ENHANCER, AND PREPARATION METHOD THEREFOR AND APPLICATION THEREOF**

(30) Priority: 28.02.2020 CN 202010129884
(71) Applicant: Wuxi Plb Therapeutics Technology Limited. Co, Wuxi, Jiangsu 214000 (CN)
(72) Inventor: DENG, Li, Wuxi, Jiangsu 214000 (CN); CHEN, Shuxian, Wuxi, Jiangsu 214000 (CN); QU, Xiuxia, Wuxi, Jiangsu 214000 (CN); GONG, Xiaohai, Wuxi, Jiangsu 214000 (CN)
(74) Representative: Santoro, Sofia
(86) International application number: PCT/CN2021/078264
(87) International publication number: WO 2021/170111

(57) **Abstract**

Provided are a tumor immune enhancer, a pharmaceutical composition thereof, and a preparation method therefor. The enhancer comprises one or more polypeptides using sequences shown in SEQ ID NOs.: 1-9 as core structures, and can be used for preparing tumor vaccines.

## Description

### Technical field

The invention relates to the field of immunity, and in particular to a tumor immune enhancer and a preparation method therefor and applications thereof.

### Background

The fundamental reason for the oncogenesis and progression of tumors is mutations in the genetic material of tissue cells that promote abnormal cell growth. Although the number of gene mutations in tumor cells can be as many as thousands, the mutated genes (driver genes) that actually initiate tumorigenesis may only involve two or three. Some driver genes have been used as targets for the design of tumor therapeutics. Studies have found that the clinical efficacy of these targeted drugs depends on the expression of other non-driver mutated genes and untargeted driver genes in tumor cells, so the clinical efficacy of these drugs is very limited. The latest research progress in tumor vaccines shows that we can use those genetic mutations that change the coding of amino acid residues to design vaccines that can cure tumors, which is an important milestone in tumor therapy.

Once the gene encoding the mutation is expressed into a protein, it may be broken down by antigen-presenting cells to produce tumor-specific antigens that can be recognized by T cells, called tumor neoantigens. Since T cells recognizing neoantigens are not restricted by central tolerance, neoantigens are considered to have strong immunogenicity and can elicit tumor-specific killer CD8+ T cell (CTL) responses. Clinical studies have confirmed the existence of such neoantigen-specific CTLs in tumor tissues, and the activity of such CTLs determines the efficacy of various tumor therapies based on, such as T cell growth factor IL-2, immune checkpoint inhibitors (PD-1 and CTLA-4 monoclonal antibody) and *in vitro* infusion of the expanded TILs.

Since most tumor neoantigens are patient-specific, vaccines against neoantigens must be customized according to the gene mutations of each patient. In addition, genetic mutations in a single tumor have polyclonal properties. To address this heterogeneity of tumors, tumor vaccines need to employ a multi-target design strategy. The number of neoantigen T epitopes used in the published clinical studies of tumor vaccines is more than 20. Only about 1/3 of neoantigens can elicit anti-tumor T cell responses through detection of CD4 and CD8 T cell responses, and more than 90% of the T cells are CD4⁺. To achieve therapeutic efficacy of tumor vaccines, it is necessary to stimulate tumor-specific CD8⁺ T cell responses. Type I helper T cells are required for activation, memory formation, and tumor infiltration of these cells. Therefore, it can be speculated that most of the neoantigens currently used in cancer vaccines are not effective in eliciting type I helper T cell responses.

One approach to enhancing type I helper T cell responses is to use exogenous T-helper polypeptides. For example, tetanus toxoid T-helper peptides and pan-DR peptides (PARDE) have been used in clinical trials. As a result, it is found that these polypeptides are ineffective to induce anti-tumor CD8+ CTL responses.

To sum up, there is still no satisfactory anti-tumor immune enhancers in the art. Therefore, there is a need in the art to develop effective immune enhancers for anti-tumor immune responses.

### Summary of the invention

The purpose of the present invention is to provide an effective immune enhancer for anti-tumor immune response and a preparation method therefor and applications thereof.

In the first aspect of the present invention, it provides a tumor immune enhancer, comprising one or more polypeptides having the structure of formula I, or a pharmaceutically acceptable salt thereof:

Z0-Z1-Z2 (I)

wherein,
Z0 is none, or a peptide segment consisting of 1-10 amino acid residues;
Z2 is none, or a peptide segment consisting of 1-10 amino acid residues;
Z1 is a peptide segment selected from the group consisting of:
   (a) a polypeptide as shown in SEQ ID NOs: 1-9;
   (b) a derivative polypeptide which is formed by substitution, deletion or addition of one, two or three amino acid residues in the amino acid sequences of SEQ ID NOs: 1-9, and which can bind to human and murine type II histocompatibility antigens.

In another preferred embodiment, the derivative polypeptide retains ≥70% of the activity of the polypeptide shown in SEQ ID NOs: 1-9 in binding to human and murine type II histocompatibility antigens.

In another preferred embodiment, the derivative polypeptide has an identity of ≥80%, preferably ≥90%, more preferably ≥95% to any of the polypeptide shown in SEQ ID NOs: 1-9.

In another preferred embodiment, the IC₅₀ of Z1 binding to the histocompatibility antigens is 5-50 nM.

In another preferred embodiment, Z0 and Z2 are not both none;

In another preferred embodiment, Z0 and Z2 contain at least 2-8, preferably 3-6 hydrophilic amino acid residues.

In another preferred embodiment, Z2 is none, and Z0 is a peptide segment consisting of 2-8, preferably 3-6 hydrophilic amino acid residues.

In another preferred embodiment, Z0 is none, and Z2 is a peptide segment consisting of 2-8, preferably 3-6 hydrophilic amino acid residues.

In another preferred embodiment, the hydrophilic amino acid is selected from the group consisting of arginine and lysine.

In another preferred embodiment, at least one of Z0 and Z2 contains (Arg)n structure, wherein n is a positive integer of 3-6.

In another preferred embodiment, the structure of formula I is a structure from N terminus to C terminus.

In another preferred embodiment, the structure of formula I is a structure from C terminus to N terminus.

In another preferred embodiment, "-" is a covalent bond (such as a peptide bond), or when Z0 is none, the "-" between Z0 and Z1 does not exist, or when Z2 is none, the "-" between Z1 and Z2 does not exist.

In another preferred embodiment, the tumor immune enhancer has an activity of enhancing tumor immunity.

In another preferred embodiment, the tumor immune enhancer can induce killer T cell responses.

In another preferred embodiment, the polypeptide having the structure of formula I comprises one or more CD4⁺Th epitope peptides.

In another preferred embodiment, the polypeptide having the structure of formula I is a CD4+ T cell antigen receptor ligand.

In another preferred embodiment, the tumor immune enhancer comprises a polypeptide having the structure of formula I wherein Z1 is shown in SEQ ID NO: 1, a polypeptide having the structure of formula I wherein Z1 is shown in SEQ ID NO: 2, or a polypeptide having the structure of formula I wherein Z1 is shown in SEQ ID NO: 3.

In the second aspect of the present invention, it provides a multimer, which is composed of m monomers in series connection, and has the function of enhancing tumor immunity, wherein, m is a positive integer ≥ 2, and each monomer independently has the structure of formula I:

Z0-Z1-Z2 (I)

wherein,
Z0 is none, or a peptide segment consisting of 1-10 amino acid residues;
Z2 is none, or a peptide segment consisting of 1-10 amino acid residues;
Z1 is a peptide segment selected from the group consisting of:
   (a) a polypeptide as shown in SEQ ID NOs: 1-9;
   (b) a derivative polypeptide which is formed by substitution, deletion or addition of one, two or three amino acid residues in the amino acid sequences of SEQ ID NOs: 1-9, and which can bind to histocompatibility antigens.

In another preferred embodiment, m is 2, 3, 4, 5 or 6.

In another preferred embodiment, the two monomers are directly connected by a peptide bond or connected by a peptide linker.

In another preferred embodiment, the peptide linker is a flexible peptide linker, a rigid peptide linker, and combinations thereof.

In another preferred embodiment, the peptide linker is a peptide linker of 3-10 amino acids.

In the third aspect of the present invention, it provides an isolated nucleic acid molecule encoding a polypeptide, wherein the polypeptide has the structure of formula I, or the polypeptide is a multimer composed of m monomers in series connection, wherein each monomer independently has the structure of formula I, and m is a positive integer ≥2.

In another preferred embodiment, the nucleic acid molecule is selected from the group consisting of: DNA, RNA, and combinations thereof.

In the fourth aspect of the present invention, it provides a pharmaceutical composition comprising:
(a) the tumor immune enhancer or a pharmaceutically acceptable salt thereof according to the first aspect of the present invention, the multimer according to the second aspect of the present invention, the isolated nucleic acid molecule according to the third aspect of the present invention, and/or the pharmaceutical composition according to the fourth aspect of the present invention; and
(b) a pharmaceutically acceptable carrier or excipient.

In another preferred embodiment, the dosage form of the composition is injection.

In another preferred embodiment, the composition is in a sustained-release dosage form.

In another preferred embodiment, the tumor immune enhancer or the pharmaceutically acceptable salt thereof according to the first aspect of the present invention, or the multimer according to the second aspect of the present invention contained in the composition can exist in a form selected from the group consisting of:
protein molecules (intact molecules, subunits, domains, polypeptides and recombinant engineered molecules), lipids, polysaccharides, and lipid and polysaccharide complexes.

In another preferred embodiment, the pharmaceutical composition further comprises:
(c) a tumor antigen.

In another preferred embodiment, the tumor antigen is a tumor neoantigen peptide.

In another preferred embodiment, the tumor antigen is an antigen that cannot effectively elicit an anti-tumor immune response by itself.

In another preferred embodiment, the tumor antigen is a natural one, a synthetic one, and combinations thereof.

In another preferred embodiment, the tumor antigen is selected from the group consisting of: short peptide, intact protein, tumor cell lysate, inactivated tumor cell, and any combination thereof.

In another preferred embodiment, the tumor antigen is from a malignant tumor with non-synonymous mutations in the codons at medium and high frequency, including but not limited to:
malignant melanoma, breast cancer, lung cancer, pancreatic cancer, prostate cancer, bowel cancer, liver cancer, esophageal cancer, cervical cancer, bladder cancer, renal cell carcinoma, and glioma multiforme.

In another preferred embodiment, the pharmaceutical composition further comprises:
(d) a DC activator.

In another preferred embodiment, the DC activator is a nano-formulation, including but not limited to w/o agent, and o/w agent.

In another preferred embodiment, the DC activator is a self-assembled nano-formulation.

In another preferred embodiment, the DC activator is selected from the group consisting of: liposome, viral capsid, and inactivated bacteria.

In another preferred embodiment, the pharmaceutical composition is a tumor vaccine composition.

In another preferred embodiment, the vaccine is selected from the group consisting of: whole cell vaccine, cell lysate vaccine, tumor tissue lysate vaccine, and tumor cell exosome vaccine.

In the fifth aspect of the present invention, it provides a use of a substance selected from the group consisting of: the tumor immune enhancer or the pharmaceutically acceptable salt thereof according to the first aspect of the present invention, the multimer according to the second aspect of the present invention, or the isolated nucleic acid molecule according to the third aspect of the present invention, wherein the substance is used for preparing a drug for improving the antitumor activity of a tumor antigen, or for preparing an antitumor vaccine (tumor vaccine) composition.

In another preferred embodiment, the pharmaceutical or vaccine composition is further used for one or more uses selected from the group consisting of:
(a) inducing a helper T cell response, preferably a type I helper T cell response;
(b) inducing a CD8+ T cell response, preferably a tumor-specific killer CD8+ T cell response;
(c) inducing a B cell response;
(d) anti-tumor cell therapy;
(e) combined use with other therapies such as immune checkpoint inhibitors.

In the sixth aspect of the present invention, it provides a kit, comprising:
(i) a first container, and the tumor immune enhancer according to the first aspect of the present invention, or a drug containing the tumor immune enhancer located in the first container;
(ii) a second container, and a tumor-treating drug located in the second container; and
(iii) a specification, in which an instruction for the combined administration of the tumor immune enhancer and the tumor-treating drug for cancer treatment is described.

In another preferred embodiment, the tumor-treating drug comprises immune checkpoint inhibitor, chemotherapeutic agent, radiotherapy agent, hyperthermia agent, oncolytic virus, and immune cell therapeutic agent.

In another preferred embodiment, the immune cell therapeutic agent comprises CAR-T cell, CAR-NK cell, TCR-T cell, DC cell, and combinations thereof.

In the seventh aspect of the present invention, it provides a method of treating cancer, comprising the steps of:
(i) administering the tumor immune enhancer or a pharmaceutically acceptable salt thereof according to the first aspect of the present invention, the multimer according to the second aspect of the present invention, the isolated nucleic acid molecule according to the third aspect of the present invention, or the pharmaceutical composition according to the fourth aspect of the present invention to a subject in need; and
(ii) administering a tumor-treating drug to the subject in need.

In another preferred embodiment, the steps (i) and (ii) can be performed simultaneously or sequentially.

In another preferred embodiment, the subject in need refers to a tumor patient.

In another preferred embodiment, in step (ii), the tumor-treating drug is administered according to a conventional administration dose and administration frequency.

In another preferred embodiment, the method is to combine existing tumor treatment methods such as immune checkpoint inhibitor, chemotherapy, radiotherapy, thermotherapy, oncolytic virus, etc. with the tumor immune enhancer of the present invention.

In another preferred embodiment, the method is to combine cell therapy with the tumor immune enhancer of the present invention. The cell therapy includes but is not limited to DC cell therapy, CAR-T cell therapy, CAR-NK cell therapy and other existing cell therapy.

In the eighth aspect of the present invention, it provides a method for preventing and/or treating a mammalian tumor, comprising a step of: administering the tumor immune enhancer or a pharmaceutically acceptable salt thereof according to the first aspect of the present invention, the multimer according to the second aspect of the present invention, the isolated nucleic acid molecule according to the third aspect of the present invention, or the pharmaceutical composition according to the fourth aspect of the present invention to a subject in need.

In another preferred embodiment, the subject is a human.

In another preferred embodiment, an antigen of the tumor is derived from a malignant tumor with medium and high frequency non-synonymous mutations in codons, such as melanoma, pancreatic cancer, prostate cancer, breast cancer, lung cancer, intestinal cancer, liver cancer, esophageal cancer, cervical cancer, bladder cancer, renal cell carcinoma, and glioma multiforme.

In another preferred embodiment, the method further comprises: administering a tumor antigen to the subject in need.

In another preferred embodiment, the method further comprises: administering a tumor-treating drug to the subject in need.

It should be understood that within the scope of the present invention, the above-mentioned technical features of the present invention and the technical features specifically described in the following (such as the embodiments) can be combined with each other to form a new or preferred technical solution, which are not redundantly repeated one by one due to space limitation.

### Description of the drawings

Figure 1 shows that in one embodiment of the present invention, UThE enhances the anti-tumor immune response of weak neoantigens in murine melanoma.
Figure 2 shows that in another embodiment of the present invention, UThE enhances the anti-tumor immune response (tumor prevention) of weak neoantigens in murine melanoma. Figures 2A and 2B show tumor volume changes as a function of days after tumor injection, respectively.
Figure 3 shows that in another embodiment of the present invention, UThE effectively enhances the therapeutic effect on murine melanoma.
Figure 4 shows the detection results of anti-UThE antibodies in mice serum samples after vaccine immunization in one embodiment.
FIG. 5 shows the detection results of anti-UThE antibodies in mice serum samples after vaccine immunization in another embodiment.
Figure 6 shows the detection results of anti-neoantigen antibodies in mice serum samples after vaccine immunization in another embodiment.
Figure 7 shows the detection results of anti-neoantigen antibodies in mice serum samples after vaccine immunization in another embodiment.
Figure 8 shows the detection results of tumor-specific CD8+ CTL in mice spleen after vaccine immunization in another embodiment.
Figure 9 shows the detection results of tumor-specific CD8+ CTLs in the spleen of immunized mice in another embodiment.
Figure 10 shows the effective killing effect of killer CD8+ CTL on tumor target cells in another embodiment.
Figure 11 shows that in another embodiment, UThE effectively enhances the immunogenicity of the weak antigen in mice lung cancer (LLC) and its tumor inhibitory effect.
Figure 12 shows that in another embodiment, UThE effectively enhances the immunogenicity of the weak antigen of mice melanoma (B16F10) and its tumor inhibitory effect.
Figure 13 shows that in another embodiment, UThE effectively enhances the immunogenicity of the weak antigen of mice prostate cancer (RM-1) and its tumor inhibitory effect.
Figure 14 shows that in another embodiment, UThE effectively enhances the immunogenicity of the weak antigen of mouse pancreatic cancer (PanC-02) and its tumor inhibitory effect.
Figure 15 shows the dose-effect relationship and safety (mice body weight was stable) of UThE on tumor cell killing in another embodiment.
Figure 16 shows the tumor inhibitory effect of a UThE candidate peptide, i.e., UThE1-7, in C57BL/6 mice and Balb/C mice in another embodiment.

### Detailed Description

After extensive and intensive research, the inventors have discovered for the first time a class of universal Th epitope peptides derived from diphtheria toxin and tetanus toxin proteins, which can enhance the specific CD8⁺CTL immune response of tumor neoantigen vaccines. Experiments show that the polypeptides of the present invention can enhance the anti-tumor immune response efficacy of tumor vaccines. On this basis, the inventors have completed the present invention.

Specifically, the present invention provides UThE1-UThE9, which are polypeptide molecules composed of 15-21 amino acid residues, and have moderate affinity with mouse type II histocompatibility antigen molecules, with IC50 of 5-50 nM. Due to the excessively strong hydrophobicity of these polypeptide molecules, multiple hydrophilic amino acid residues (such as arginine residues or lysine residues) are required to be added at the N terminus or C terminus thereof during synthesis. These polypeptide molecules all carry one or more Th epitopes, which can support the function of inducing helper T cell responses in more than 80% of the population.

### Active polypeptide

In the present invention, the terms "polypeptide of the present invention", "UThE polypeptide", "Th epitope peptide" or "UThE1-UThE9" can be used interchangeably, and all refer to the polypeptide having the structure of formula I:

Z0-Z1-Z2 (I)

wherein,
Z0 is none, or a peptide segment consisting of 1-10 amino acid residues;
Z2 is none, or a peptide segment consisting of 1-10 amino acid residues;
Z1 is a peptide segment selected from the group consisting of:
   (a) a polypeptide as shown in SEQ ID NOs: 1-9;
   (b) a derivative polypeptide which is formed by substitution, deletion or addition of one, two or three amino acid residues in the amino acid sequences of SEQ ID NOs: 1-9, and which can bind to type II histocompatibility antigens.

The polypeptide of the present invention includes variant forms of the sequences of SEQ ID NOs: 1-9 with the function of enhancing tumor immunity. These variant forms include (but are not limited to): deletion, insertion and/or substitution of 1-4 (preferably 1-3, more preferably 1-2, most preferably 1) amino acids, and addition or deletion of one or more (usually within 4, preferably within 3, more preferably within 2) amino acids at C terminus and/or N terminus. For example, in the art, substitution with amino acids of parallel or similar properties generally does not alter the function of the protein. For another example, addition or deletion of one or several amino acids at the C terminus and/or N terminus usually does not change the structure and function of the protein. Furthermore, the term also includes monomeric and multimeric forms of the polypeptide of the invention. The term also includes linear as well as nonlinear polypeptides (e.g., cyclic peptides).

Due to the excessively strong hydrophobicity of the polypeptide molecule of the present invention, multiple hydrophilic amino acid residues (such as arginine residues or lysine residues) are required to be added at the N terminus or C terminus thereof during synthesis. A typical polypeptide of the present invention is synthesized by adding 3-6 arginine to the N terminus or C terminus of the polypeptides shown in SEQ ID NOs: 1-9.

The present invention also includes active fragments, derivatives and analogs of a UTHE polypeptide. As used herein, the terms "fragment," "derivative," and "analog" refer to a polypeptide that substantially retains the function or activity of enhancing tumor immunity. The polypeptide fragment, derivative or analog of the present invention may be (i) a polypeptide in which one or more conservative or non-conservative amino acid residues (preferably conservative amino acid residues) are substituted, or (ii) a polypeptide with a substitution group in one or more amino acid residues, or (iii) a polypeptide formed by fusion of a DTHE polypeptide with another compound (such as a compound that prolongs the half-life of the polypeptide, such as polyethylene glycol), or (iv) a polypeptide formed by fusion of additional amino acid sequence to this polypeptide sequence (a protein formed by fusion with a leader sequence, a secretory sequence or a tag sequence such as 6His). According to the teachings herein, these fragments, derivatives and analogs fall within the scope which is well known to those skilled in the art.

A preferred class of active derivatives refers to polypeptides in which up to 4, preferably up to 3, more preferably up to 2, and most preferably 1 amino acid(s) are(is) replaced by amino acids of similar or close properties compared to the amino acid sequence of formula I. These conservatively variant polypeptides are preferably generated by amino acid substitutions according to Table A.

**Table A**

| Initial residues | Representative substitution | Preferred substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

The present invention also provides analogs of DTHE polypeptides. The differences between these analogs and native DTHE polypeptides can be differences in amino acid sequence, differences in modification that does not affect the sequence, or both. Analogs also include analogs with residues other than natural L-amino acids (e.g., D-amino acids), as well as analogs with non-naturally occurring or synthetic amino acids (e.g., β, γ-amino acids). It should be understood that the polypeptide of the present invention is not limited to the representative polypeptides exemplified above.

Modification (usually not changing the primary structure) includes: chemically derived forms of the polypeptide *in vivo* or *in vitro,* such as acetylation or carboxylation. Modifications also include glycosylation, such as those polypeptides produced by glycosylation modification during the synthesis and processing of the polypeptide or during further processing steps. This modification can be accomplished by exposing the polypeptide to an enzyme that performs glycosylation (such as a mammalian glycosylase or deglycosylase). Modification also includes sequences having phosphorylated amino acid residues (such as phosphotyrosine, phosphoserine, phosphothreonine). It also includes polypeptides that have been modified to increase their resistance to proteolysis or to optimize their solubility.

The polypeptide of the present invention may also be used in the form of salts derived from pharmaceutically or physiologically acceptable acids or bases. These salts include (but are not limited to) salts formed with the following acids: hydrochloric acid, hydrobromic acid, sulfuric acid, citric acid, tartaric acid, phosphoric acid, lactic acid, pyruvic acid, acetic acid, succinic acid, oxalic acid, fumaric acid, maleic acid, oxaloacetic acid, methanesulfonic acid, ethanesulfonic acid, benzenesulfonic acid, or isethionic acid. Other salts include those formed with alkali metals or alkaline earth metals (such as sodium, potassium, calcium or magnesium), and those in the form of esters, carbamates or other conventional "prodrugs".

### Tumor immune enhancer

The present invention provides a tumor immune enhancer, which comprises one or more polypeptides having the structure of formula I.

The tumor immune enhancer of the invention can enhance the specific CD8⁺ CTL immune response of tumor neoantigen vaccines, and has the activity of enhancing the anti-tumor immune response of the tumor vaccines.

In a preferred embodiment, the tumor immune enhancer of the present invention comprises the polypeptides shown in SEQ ID NO.: 1-3 or their derivative polypeptides.

### Tumor neoantigen polypeptide

The protein with specific amino acid sequence variation produced by cancer cells on the basis of gene variation is called "neoantigen" (NeoAg). This is because without the changes of amino acid sequence, these proteins should be non-antigenic. Once mutated, these proteins will then attract the attention of autoimmune cells and trigger a series of immune responses.

The tumor immune enhancer of the present invention can enhance the immune response of tumor neoantigens, especially the immune response against an antigen that cannot effectively elicit anti-tumor immune response. The tumor antigens can be natural, synthetic, and combinations thereof. The tumor antigens can be: short peptides, intact proteins, tumor cell lysates, and combinations thereof.

In a preferred embodiment, four different melanoma cell B16F10 neoantigen peptides (NeoAgs) are used as vaccines, which do not produce any antigen-specific anti-tumor CTLs when administered alone.

The cell lysate according to the present invention is the supernatant obtained by suspending cells in an equal volume of phosphate buffer, breaking cells by freeze-thaw method, and centrifuging at 10,000g for 10 minutes to remove the precipitate, thereby obtaining a supernatant as the cell lysate.

### Coding sequence

The present invention also relates to a polynucleotide encoding a DTHE polypeptide. A preferred coding sequence encodes the short peptides shown in SEQ ID NOs: 1-9.

The polynucleotide of the present invention may be in the form of DNA or RNA. DNA can be the coding or non-coding strand. The full-length sequence of the polynucleotide of the present invention or its fragment can usually be obtained by PCR amplification method, recombinant method or artificial synthesis method. At present, DNA sequence encoding the polypeptide of the present invention (or fragments thereof, or derivatives thereof) can be obtained entirely by chemical synthesis. This DNA sequence can then be introduced into various existing DNA molecules (or e.g. vectors) and cells known in the art.

The present invention also relates to a vector comprising the polynucleotide of the present invention, and a host cell genetically engineered with the vector or the UTHE polypeptide coding sequence of the present invention.

### Preparation of UThE polypeptide

The polypeptide of the present invention may be a recombinant polypeptide or a synthetic polypeptide. The polypeptide of the present invention may be chemically synthesized, or recombinant. Correspondingly, the polypeptide of the present invention can be artificially synthesized by conventional methods or produced by recombinant methods.

In a preferred embodiment, the UThE polypeptide with 6 Arg at the end can be synthesized by chemical synthesis, the HPLC purification yield is greater than 20%, and the purity is greater than 99.9%.

A preferred method is to use liquid-phase synthesis techniques or solid-phase synthesis techniques such as Boc solid-phase method, Fmoc solid-phase method or a combination of both methods. Solid-phase synthesis can quickly obtain samples, and an appropriate resin carrier and synthesis system can be selected according to the sequence characteristics of the target peptide. For example, the preferred solid-phase carrier in the Fmoc system is Wang resin, which is linked to the C-terminal amino acid in the peptide, the structure of the Wang resin is polystyrene, and the arm between the Wang resin and the amino acid is 4-alkoxybenzyl alcohol. After treatment with 25% hexahydropyridine/dimethylformamide at room temperature for 20 minutes to remove the Fmoc protecting group, and the amino acid from C terminus to N terminus is extended one by one according to the given amino acid sequence. After the synthesis, the synthesized proinsulin-related peptide will be cleaved from the resin with trifluoroacetic acid containing 4% *p*-cresol and the protective group is removed. The crude peptide can be obtained by filtration and ether precipitation after removing the resin. After the resulting product solution is lyophilized, the desired peptide is to be purified by gel filtration and reversed-phase high pressure liquid chromatography. When using the Boc system for solid-phase synthesis, the preferred resin is PAM resin, which is linked to the C-terminal amino acid in the peptide, the structure of the PAM resin is polystyrene, and the arm between the PAM resin and the amino acid is 4-hydroxyphenylacetamide. In the Boc synthesis system, the protecting group Boc is removed with TFA/dichloromethane (DCM) and neutralized with diisopropylethylamine (DIEA/dichloromethane) in a cycle of deprotection, neutralization, and coupling. After the peptide chain condensation is completed, the peptide chain is cleaved from the resin and the protecting group is removed at the same time by treating with hydrogen fluoride (HF) containing *p*-cresol (5-10%) at 0 °C for 1 hour. The peptide is extracted with 50-80% acetic acid (containing a small amount of mercapto-ethanol). After the solution is lyophilized, it is further separated and purified by molecular sieve Sephadex G10 or Tsk-40f, and then purified by high pressure liquid phase to obtain the desired peptide. Each amino acid residue can be coupled using a variety of coupling agents and coupling methods known in the art of peptide chemistry, such as dicyclohexylcarbodiimide (DCC), hydroxybenzotriazole (HOBt) or 1,1,3,3-tetramethyluronium hexafluorophosphate (HBTU) for direct coupling. The purity and structure of the synthesized short peptides can be confirmed by reversed-phase high performance liquid chromatography and mass spectrometry analysis.

Another approach is to use recombinant techniques to produce the polypeptide of the present invention. By using conventional recombinant DNA technology, the polynucleotide of the present invention can be used to express or produce a recombinant DTHE polypeptide. Generally, it comprises the following steps:
(1) using the polynucleotide (or variant) of the present invention encoding the DTHE polypeptide, or using a recombinant expression vector containing the polynucleotide to transform or transfect a suitable host cell;
(2). culturing the host cell in a suitable medium;
(3). separating and purifying the protein from the medium or cells.

The recombinant polypeptide can be expressed in the cell or on the cell membrane, or secreted out of the cell. If necessary, its physical, chemical and other characteristics can be used to separate and purify the recombinant protein by using various separation methods. These methods are well known to those skilled in the art. Examples of these methods include, but are not limited to: conventional renaturation treatment, treatment with protein precipitation agents (salting out method), centrifugation, breaking bacteria via osmosis, ultra-treatment, ultra-centrifugation, molecular sieve chromatography (gel filtration), adsorption chromatography, ion exchange chromatography, high performance liquid chromatography (HPLC) and various other liquid chromatography techniques and combinations thereof.

Since the polypeptide of the present invention is relatively short, it may be considered to connect multiple polypeptides in series, and after recombinant expression, the expression product in the form of a multimer can be obtained, and then the desired small peptide can be formed by methods such as enzyme digestion.

### Pharmaceutical composition and administration method

The present invention also provides a pharmaceutical composition, which may be therapeutic or prophylactic (e.g., a vaccine). The pharmaceutical composition of the present invention comprises (a) a safe and effective dose of the polypeptide of the present invention or a pharmaceutically acceptable salt thereof; and (b) a pharmaceutically acceptable carrier or excipient.

For the purposes of the present invention, an effective dose is about 10 µg (microgram)-100 mg/dose, preferably 100-1000 µg/dose, of the polypeptide of the invention administered to an individual. In addition, the polypeptide of the present invention can be used alone or in combination with other therapeutic agents (e.g., formulated in the same pharmaceutical composition).

In the present invention, the prophylactic pharmaceutical composition may be a vaccine composition, which comprises the polypeptide of the present invention and a tumor antigen, and is usually combined with a "pharmaceutically acceptable carrier".

The term "pharmaceutically acceptable carrier" refers to a carrier for administration of a therapeutic agent. The term refers to pharmaceutical carriers which do not themselves induce the production of antibodies detrimental to the individual receiving the composition, and are not unduly toxic after administration. These carriers are well known to those skilled in the art. A thorough discussion of pharmaceutically acceptable excipients can be found in Remington's Pharmaceutical Sciences (Mack Pub. Co., N.J. 1991). Such carriers include (but are not limited to): saline, buffers, dextrose, water, glycerol, ethanol, adjuvants, and combinations thereof. In addition, auxiliary substances such as wetting or emulsifying agents, pH buffering substances and the like may also be present in these carriers.

Furthermore, the (vaccine) composition of the present invention may also contain additional adjuvants. Representative vaccine adjuvants include (but are not limited to) the following classes: inorganic adjuvants, such as aluminum hydroxide, alum, etc.; synthetic adjuvants, such as artificially synthesized double-stranded polynucleotides (double-stranded polyadenylic acid, double-stranded polyuridinic acid), levamisole, isoproinosine, etc.; oil agents, such as Freund's adjuvant, peanut oil emulsion adjuvant, mineral oil, vegetable oil, etc.

Adjuvants also include various novel adjuvants and adjuvant components that are believed to be useful in vaccine adjuvants in current and subsequent studies.

In general, vaccine compositions or immunogenic compositions can be prepared as injection, such as liquid solution or suspension. Solid form can also be prepared and solution or suspension, liquid excipients are added prior to injection. The formulation can also be emulsified or encapsulated in liposomes to enhance adjuvant effects.

The compositions can be formulated in unit or multi-unit dosage form. Each dosage form contains a predetermined amount of active substance calculated to produce the desired therapeutic effect, together with suitable pharmaceutical excipients.

Once formulated, the composition of the present invention may be administered by conventional routes including (but not limited to): intravenous, intratumoral, intramuscular, intraperitoneal, subcutaneous, intradermal, paracancerous, or topical administration. The subject to be prevented or treated can be an animal; especially a human.

When the composition of the present invention is used for actual treatment, various pharmaceutical compositions in different dosage forms can be adopted according to the usage situation. These pharmaceutical compositions can be formulated according to conventional methods by mixing, diluting or dissolving, with occasional addition of suitable pharmaceutical additives such as excipients, disintegrants, binders, lubricants, diluents, buffers, isotonicities, preservatives, wetting agents, emulsifiers, dispersants, stabilizers and solubilizers, and the formulation process can be carried out in a conventional manner according to the dosage form.

The pharmaceutical composition of the present invention can also be administered in the form of sustained release formulations. For example, the short peptide DTHE or a salt thereof can be incorporated into a pellet or microcapsule supported by a slow-release polymer, and the pellet or microcapsule is then surgically implanted into the tissue to be treated. As examples of sustained-release polymers, ethylene-vinyl acetate copolymer, polyhydrometaacrylate, polyacrylamide, polyvinylpyrrolidone, methylcellulose, polylactic acid, poly lactic-co-glycolic acid and the like can be exemplified, and preferably biodegradable polymers such as polylactic acid and poly lactic-co-glycolic acid can be exemplified .

When the pharmaceutical composition of the present invention is used for actual treatment, the dose of the short peptide DTHE or a pharmaceutically acceptable salt thereof as an active ingredient can be reasonably determined according to the weight, age, sex, and severity of symptoms of each patient to be treated.

The present invention includes the following main advantages:
(a) The present invention utilizes composite antigen carrier technology to identify tumor neoantigens to the greatest extent;
(b) The purity and quality of the vaccine prepared by using the polypeptide of the present invention are easier to control, with high safety and less toxic side effects;
(c) The human immune response rate is high and more efficient;
(e) The polypeptide of the present invention can improve the activation efficiency of immune cells *in vitro.*

The present invention will be further illustrated below with reference to the specific examples. It should be understood that these examples are only to illustrate the invention, not to limit the scope of the invention. The experimental methods with no specific conditions described in the following examples are generally performed under the conventional conditions e.g., the conditions described by Sambrook et al., Molecular Cloning: Laboratory Guide (New York: Cold Spring Harbor Laboratory Press, 1989), or according to the manufacture's instructions. Unless indicated otherwise, all percentage and parts are calculated by weight.

### Preparation Example 1 Synthesis of UThE

In this preparation example, the chemical synthesis method was used to synthesize the UThE polypeptides whose amino acid sequences were shown in the following table, and wherein the combination of #1, #2, and #3 was used in the following example.

| Name | Amino acid sequence | SEQ ID NO. |
|---|---|---|
| UThE1 | LSELKTVTGTNPVFAGANYAAWAV | 1 |
| UThE2 | TGTNPVFAGANYAAWAVNVAQVID | 2 |
| UThE3 | SIALSSLMVAQAIPLVGELVDIGFAAY | 3 |
| UThE4 | IT AENTPLPIAGVLLPTIPGKLD | 4 |
| UThE5 | TTAALSILPGIGSVMGIADGAV | 5 |
| UThE6 | IVAQSIALSSLMVAQAIPLVGELV | 6 |
| UThE7 | GELVDIGFAAYNFVESIINLFQVV | 7 |
| UThE8 | TNSVDDALINSTKIYS | 8 |
| UThE9 | KAIHLVNNESSEVIVH | 9 |

### Preparation Example 2 Preparation of B16F10 tumor neoantigen peptides

In this preparation example, a chemical synthesis method was used to synthesize neoantigen peptides (neoAg) with the following amino acid sequences:
The amino acid sequences of the four neoantigen peptides (neoAg) were as follows:

| neoAg Name | Amino acid sequence | SEQ ID No. |
|---|---|---|
| Pi4k2b | WLPQAKVPFSEETQNLILPYISDMNFV | 10 |
| ddb1 | LVLSFVGQTRVLMINGEEVEETELMGF | 11 |
| Pcdhga11 | RGQSQLFSLNPRGRSLVTAGRIDREEL | 12 |
| Atp11a | SSPDEVALVEGVQSLGFTYLRLKDNYM | 13 |

### Preparation Example 3 Preparation of tumor cell lysates

The cells were suspended in an equal volume of phosphate buffer, freeze-thawed to break the cells, and centrifuged at 10,000 g for 10 minutes to remove the precipitate and the supernatant was used as a cell lysate. As tumor antigens, B16F10 cell lysates, LLC cell lysates, and Hepa1-6 cell lysates were prepared, respectively.

### Example 1 UThE enhances the anti-tumor immune response of weak neoantigens in melanoma

Four neoantigen peptides (neoAg) from the B16F10 cell line which had shown to be ineffective to induce antitumor immune responses in mice in preclinical studies were selected. The B16F10 cell line referred to mice melanoma cells.

The four neoAg peptides (#1, #2, #3 and #4) were mixed in a weight ratio of 1:1:1:1 and formulated into a vaccine together with a UThE peptide and adjuvant. After immunizing mice, the anti-tumor effect was tested. The specific method was as follows:
C57BL6 mice (6-week aged, 7 in each experimental group) were immunized three times with a one-week interval. 200 microliters of vaccine was used per immunization per mouse, and 50 microliters were injected subcutaneously at lateral sites adjacent to the extremities. The dosage of each component of 200 µl vaccine was 50 µg of neoAg, 50 µg of DThE, 300 µg of Alum and 20 µg of CpG. The vaccine injection was prepared with PBS. The experimental groups were: (1) PBS group (Alum+CpG+PBS); (2) neoAg group (neoAg+Alum+CpG+PBS); (3) neoAg+UThE group (neoAg+UThE+Alum+CpG+PBS). On the third day after the third immunization, 10⁵ B16F10 cells were subcutaneously inoculated near the right armpit of the mice. Cells were suspended in 100 microliters of PBS. The tumor growth was then observed and recorded.

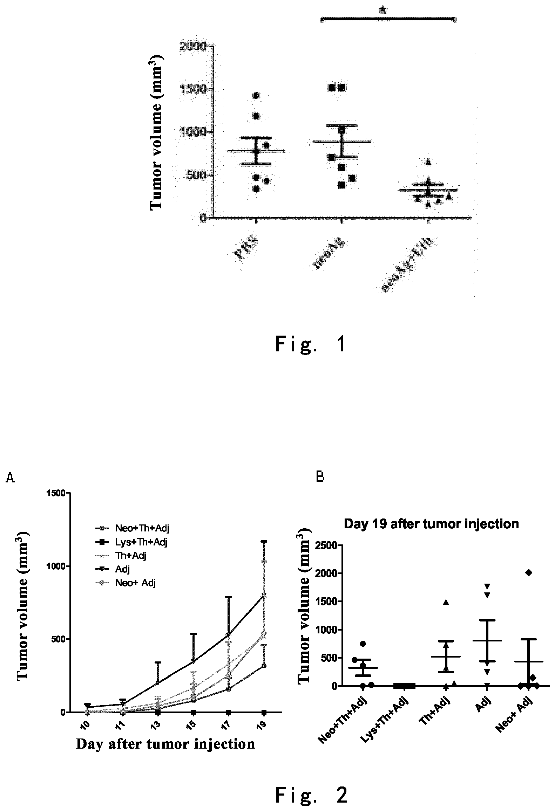

The results were shown in Figure 1 and Table 1. On day 7 after inoculation, the mice started to develop tumors. On the 15th day, the mean tumor volume (mean±SEM) of mice in PBS, NeoAg, and NeoAg+UThE groups were 785±153 mm³, 890±80 mm³, and 328±65 mm³, respectively.

**Table 1**

| Group | Administered Substance | Tumor volume (15d) (mean+SEM, mm³) | Relative tumor volume (%) |
|---|---|---|---|
| 1 | Alum+CpG+PBS | 785±153 | 100% |
| 2 | neoAg+Alum+CpG+PBS | 890±80 | 113% |
| 3 | neoAg+UThE+Alum+CpG +PBS | 328±65 | 41.7% |

The results showed that administration of neoAg and adjuvant (Alum+CpG) did not inhibit tumor formation in NeoAg group compared with PBS group. In the NeoAg+UTh group, tumor growth was significantly inhibited.

### Example 2 UThE enhances the anti-tumor immune response of weak neoantigens in melanoma

The method of this example was basically the same as that of Example 1 except the preparation of the vaccine and the number of immunizations, and B16F10 cell lysate or neoAg as the tumor antigen. The Method was as below:
C57BL/6 mice (6-week aged, 5 in each experimental group) were immunized four times with a one-week interval. Each mouse was injected with the vaccine at four lateral sites near the extremities, with 50 microliters per site. The dosage of each 200 microliters of vaccine components in the first and second immunizations was 25 µg of neoAg or B16F10 cell lysate, 25 µg of UThE, 25 µg of adjuvant (adj), and 100 microliters of MF59. The adjuvant included 12.5 µg CpG, 12.5 µg of PolyI:C, and was formulated in PBS. For the third and fourth immunizations, the dosage of each 200 microliter vaccine component was 12.5 µg of neoAg or B16F10 cell lysate, 12.5 µg of UThE, 12.5 µg of adjuvant (adj), and 59 microliters of MF. The adjuvant included 6.25 µg of CpG, 6.25 µg of PolyI:C, and was formulated in PBS. The administration of each group was as follows:
On the third day after the fourth immunization, 10⁵ B16F10 cells were subcutaneously inoculated near the right armpit of the mice. Cells were suspended in 100 microliters of PBS. The tumor growth was then observed and recorded. Tumor volume was measured every other day after tumors were visible.

The results were shown in Figure 2 and Table 2. From the injection of tumor cells to the 19th day, the order of tumor growth speed and size was: adj group>UThE+adj group>neo+adj group>neo+UThE+adj group>Lys+UThE+Adj group. Among them, the mice in the Lys+UThE+Adj group still did not develop tumors on the 19th day. In addition, the mice in the Neo+UThE+Adj group also had smaller tumors and slower tumor growth (Fig. 2A).

On day 19, the mean tumor volume (mean±SEM) of adj group, UTEh+adj group, neo+adj group, neo+UThE+adj group and Lys+UThE+Adj group were 801.1+821.7 mm³, 517.4 ±615.5 mm³, 431.8±886.7 mm³, and 317.8±314.4 mm³, respectively (Figure 2B).

**Table 2**

| Group | Administered Substance | Tumor volume (19d) (mean+SEM, mm³) | Relative tumor volume (%) |
|---|---|---|---|
| Adj | CpG+PolyI:C | 801.1±821.7 | 100% |
| Th+adj | UThE+CpG+Polyl:C | 517.4±615.5 | 64.5% |
| Neo+adj | neoAg+CpG+PolyI:C | 431.8±886.7 | 53.9% |
| Neo+Th+adj | DThE+neoAg+CpG+PolyI:C | 317.8±314.4 | 39.7% |
| Lys+Th+adj | Lysate+CpG+PolyI:C | 0 | 0% |

The above results showed that when the antigen peptide (NeoAg or B16F10 cell lysate), which was otherwise not immunoreactive, was administered together with the UThE peptide, the tumor growth was significantly inhibited in the vaccinated mice, and tumor growth could be completely inhibited when tumor cell lysate+UThE peptide was used.

### Example 3 UThE effectively enhances the therapeutic effect on melanoma B16F10

In Examples 1-3, it has been confirmed that pre-administration of UThE peptide can act as an immune enhancer, enhancing the antitumor effect of the tumor antigen peptide. In this example, it was further verified whether administration of the tumor immune enhancer UThE peptide in tumor-bearing mice could enhance the therapeutic effect on tumors.

The method was as follows: simulating tumor treatment by inoculating tumor cells first, and then treating with UThE immune enhancer.

1×10⁵ B16F10 cells were inoculated into the right forelimb of mice near the armpit, and the mice were immunized 5 days later. Each mouse was inoculated with 100 microliters of vaccine, and 50 microliters per site were injected at the injection sites near the hind limbs on both sides. The dosage of each component of the vaccine per 200 microliters was: 25 µg of UThE, adjuvant Adj, and 100 microliters of MF59. The adjuvant included 12.5 µg of CpG, 25 µg of PolyI:C, and was formulated in PBS. A total of four immunizations were performed, each with a one-week interval. After tumors began to appear, tumor size was measured and the mice were weighed.

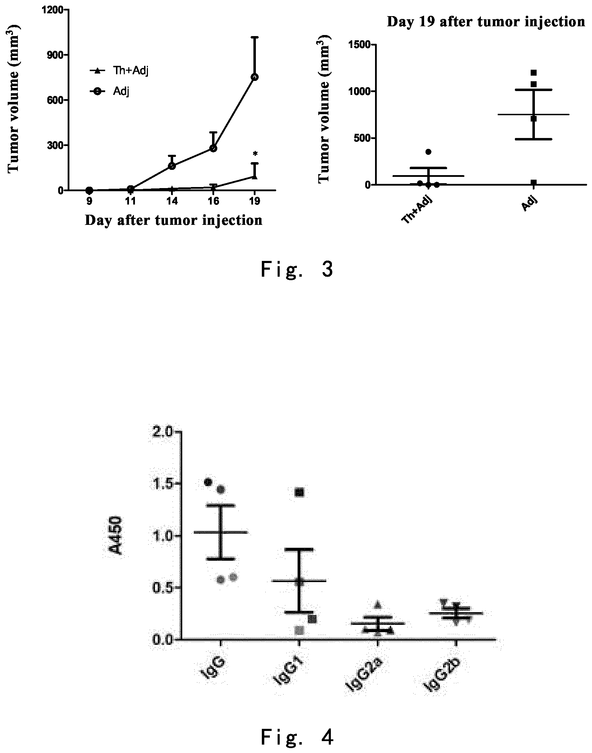

The results were shown in Figure 3 and Table 3.

**Table 3**

| Group | Administered Substance | Tumor volume (19d) (mean±SEM, mm³) | Relative tumor volume (%) |
|---|---|---|---|
| Adj | adj | 752.0±264.4 | 100% |
| Th+adj | UThE+adj | 92.6±87.05 | 12.3% |

The results showed that in tumor-bearing mice, administration of UThE could effectively enhance the cellular immunity of mice and inhibit the growth of tumor cells.

### Example 4 UThE enhances the humoral immune response of NeoAg as detected by ELISA

The immunized serum of the mice was diluted with 0.1% BSA in PBS at a volume ratio of 1:100. 100 µl of neoantigen peptide solution (10 µg/ml, pH 9.5, carbonic acid buffer) was added to the 96-well plate and coated overnight at 4°C. Then, the 96-well plate was blocked with 0.1% BSA in PBS for 2 hours at room temperature. After removing the blocking solution, 100 microliters of diluted serum were added and then incubated at room temperature for 1 hour. The serum was then removed, and the 96-well plate was washed with 0.05% Tween-20 PBS three times (incubated for 5 minutes each time). 1:5,000 diluted HRP-conjugated goat anti-mouse IgG, IgG1, IgG2a, IgG2b were added, incubated at room temperature for 1 hour and then the antibody solution was removed. The 96-well plate was washed three times with 0.05% Tween-20 PBS and once with redistilled water. TMB color development was carried out according to the instructions, and the absorbance at 450 nm was read on a microplate reader.

The results were shown in Figures 4-7.

Figure 4 showed the results of ELISA detection of UThE antibodies in serum samples of immunized mice. Mice were immunized with DThE+CpG+polyI:C+MF59 vaccine four times, each time with a one-week interval, and 7.5×10⁴ B16F10 cells were inoculated 10 days after immunization. When the tumor volume of the mice reached 1500 mm³ or when the experiment reached the 30th day after tumor inoculation, the mice were sacrificed and blood was collected. Since IgG1 and IgG2 coexisted and their concentration ratio was greater than 1, the T helper cell response was a mixed type of type I + type II, and the type II response was stronger.

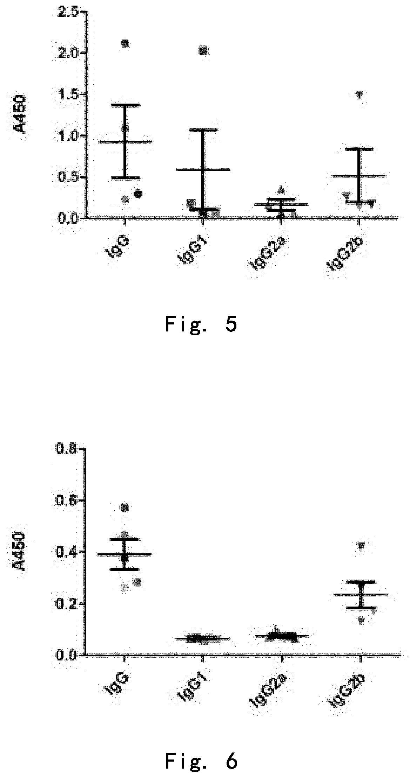

Figure 5 showed the results of ELISA detection of UThE antibodies in serum samples of immunized mice. Mice were immunized with neoAg+UThE+CpG+ polyI:C+MF59 vaccine four times, each time with a one-week interval, and 7.5×10⁴ B16F10 cells were inoculated 10 days after immunization. When the tumor volume of the mice reached 1500 mm³ or when the experiment reached the 30th day after tumor inoculation, the mice were sacrificed and blood was collected. Since IgG1 and IgG2 coexisted and their concentration ratio was greater than 1, the T helper cell response was a mixed type of type I + type II, and the type II response was stronger.

Figure 6 showed the results of ELISA detection of neoAg antibodies in serum samples of immunized mice. Mice were immunized with neoAg+CpG+ polyI:C+MF59 vaccine four times, each time with a one-week interval, and 7.5×10⁴ B16F10 cells were inoculated 10 days after immunization. When the tumor volume of the mice reached 1500 mm³ or when the experiment reached the 30th day after tumor inoculation, the mice were sacrificed and blood was collected. The antibodies produced were predominantly IgG2 and, therefore, the T helper cell response was type I.

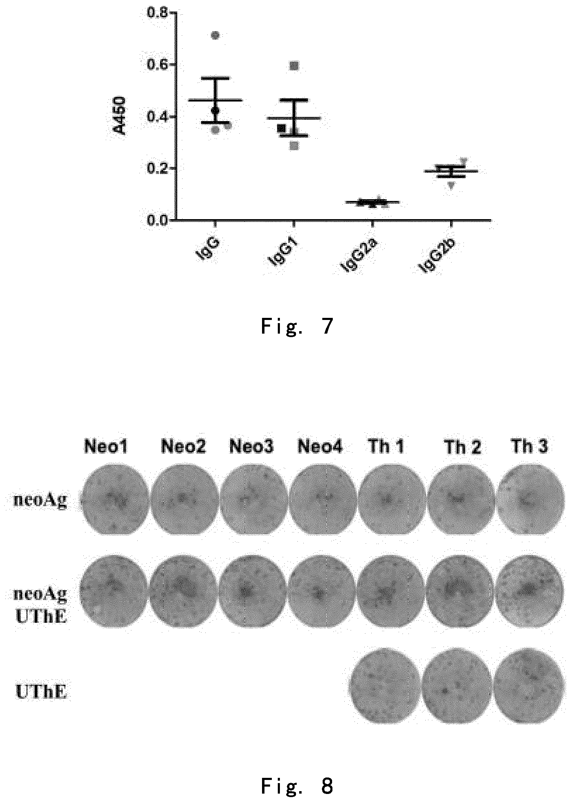

Figure 7 showed the results of ELISA detection of neoAg antibodies in serum samples of immunized mice. Mice were immunized with neoAg+CpG+ polyI:C+MF59 vaccine four times, each time with a one-week interval, and 7.5×10⁴ B16F10 cells were inoculated 10 days after immunization. When the tumor volume of the mice reached 1500 mm³ or when the experiment reached the 30th day after tumor inoculation, the mice were sacrificed and blood was collected. Since IgG1 and IgG2 coexisted and their concentration ratio was greater than 1, the T helper cell response was a mixed type of type I + type II, and the type II response was stronger.

The results in Figures 4 to 7 showed that the UThE peptide, as a tumor immune enhancer, could significantly enhance the humoral immune response against tumor neoantigens.

### Example 5 UThE polypeptides promote neo-Ag specific CD8+ CTL cell response

In this example, it was verified whether UThE polypeptides could promote neoAg-specific CTL cell responses.

The method was as follows: lymphocytes from spleen of the immunized mice were incubated with UThE or neoAg antigen-presenting cells for 5 days, and were then purified using T lymphocytes or CD8+ T Isolation Kit. 100 ml of culture medium containing 10⁴ purified lymphocytes was added to the immunospot 96-well plate coated with INF-γ capture antibody, and then incubated for 16 hours at 37°C, with 5% CO₂. Afterwards, the number of INF-γ-secreting T cells was detected by immunospot kit. After T effector cells were co-cultured with tumor target cells at a ratio of 20:1, LDH assay was performed to detect cell killing.

Preparation method of UThE or neoAg antigen-presenting cells (dendritic cells): after euthanasia of healthy mice of the same strain, the skin of the mice was separated, the front and rear leg bones were taken, and the two ends of the leg bones were cut off with scissors. The 1 ml syringe with PBS (containing 1% FBS) was used, the needle was pointed at the red dot at one end of the leg bones, and the bone marrow cells were washed out with PBS. The tissue block was gently dispersed with a pipette, and transferred into a 50 ml centrifuge tube after passing through a 200-mesh cell strainer, and centrifuged at 400 g for 10min. The cells were resuspended with PBS, added with red blood cell lysate, and centrifuged at 400 g for 15min. After cell counting, the cells were seeded into 24 well plates (most preferably 5×10⁵-1×10⁶ cells/ml). IL-4 was added to maintain the culture for 7 days, 25 ng/ml TNFα was added, and 10 µg/ml corresponding antigens (UThE and NeoAg) were added after 4h.

Preparation method of mice spleen lymphocytes: the spleen of mice in the experimental group was taken out and ground into pieces. Then the spleen pieces were washed through a 200-mesh cell strainer with the culture medium, and after centrifugation at 400g for 8min, the supernatant was removed. The erythrocytes were removed with red blood cell lysis buffer, the cells were resuspended in PBS and counted, and IL-2 (20 ng/ml) was added into the medium to continue the culture.

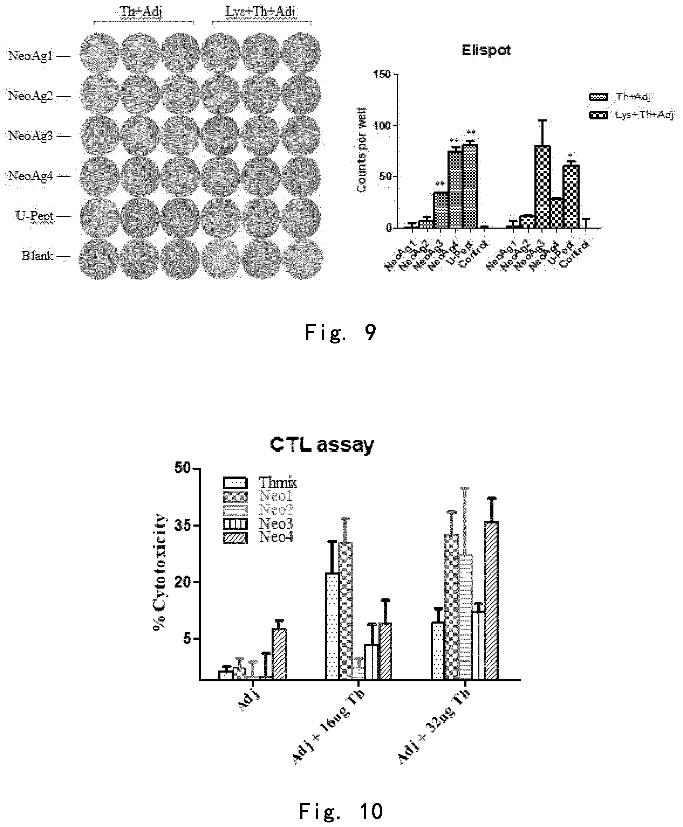

The results of the ELIspot experiments were shown in Figure 8 and Figure 9. The killing effect of CTL effector cells on tumor target cells (20:1) was shown in Figure 10. The results showed that the UThE peptide, as a tumor immune enhancer, could significantly enhance the tumor neoantigen-specific CD8+ CTL cell response, so it could effectively enhance the immune effect of tumor vaccines.

### Example 6 UThE enhances the anti-tumor immune response of tumor lysates

The method of this example was basically the same as that of Example 1 and Example 2, except that on the basis of the experiment of the original melanoma cell B16F10, the lung cancer cell LLC, the prostate cancer cell RM-1, and the pancreatic cancer cell PanC-02 were added. The preparation and immunization times of the vaccine were basically the same, by using tumor antigens from lysates of corresponding cells. Method was as follows:
C57BL/6 mice (6-week aged, 4-6 in each experimental group) were immunized four times with a one-week interval. The vaccine was injected subcutaneously at 2 sites at the root of the hind limbs of each mouse, with 50 microliters per site. The four immunization doses were the same. The dosage of vaccine components per 50 µl was 25 µg cell lysate, 25 µg UThE, and adjuvant, wherein the adjuvant comprised 25 µl MF59, 25 µg CpG and 12.5 µg PolyI:C, and was formulated in PBS.

The administration of each group was as follows:
On the third day after the fourth immunization, the mice were inoculated subcutaneously near the right armpit, wherein the inoculation amount of B16F10 cells was 7.5×10⁴, the inoculation amount of LLC cells was 1×10⁵, and the inoculation amount of RM-1 cells was 1×10⁵. Cells were suspended in 100 microliters of PBS. The tumor growth was then observed and recorded. Tumor volume was measured every other day after tumors were visible.

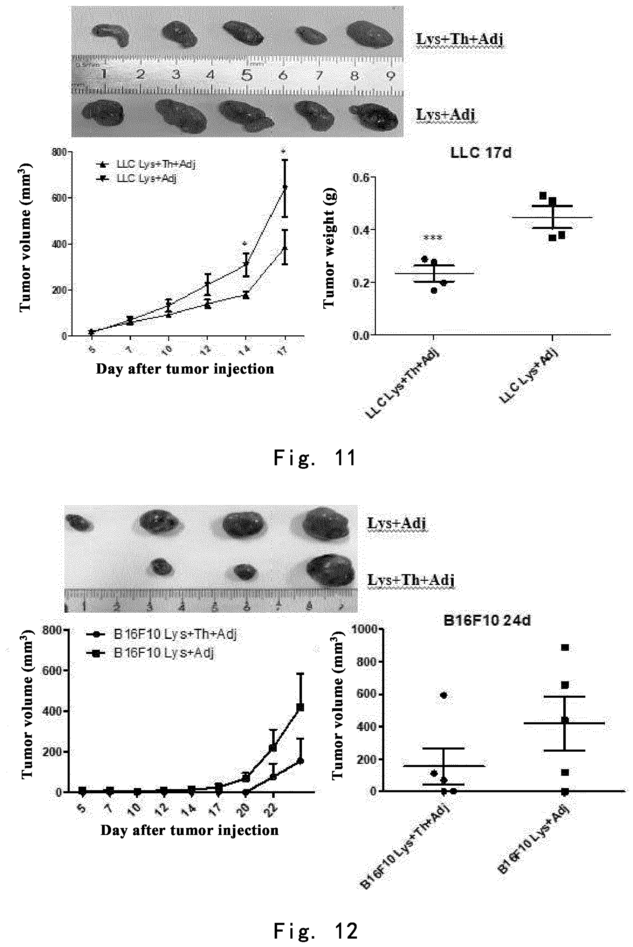

The results of lung cancer LLC cell inoculation were shown in Figure 11 and Table 4. From the injection of tumor cells to the 17th day, the order of tumor growth speed and size was: Lys+adj group>Lys+Th+Adj group. The size of the tumors on the 17th day was shown in Figure 11, and the mean tumor weight (mean±SEM) was 0.4475±0.04211 g and 0.235±0.02958 g, respectively.

**Table 4**

| Group | Administered Substance | Tumor weight (17d) (mean+SEM, g) | Relative tumor weight (%) |
|---|---|---|---|
| Lys+Adj | LLC Lys+adj | 0.4475±0.04211 | 100% |
| Lys+Th+adj | LLC Lys+Th+adj | 0.235±0.02958 | 52.5% |

The results of melanoma B16F10 cell inoculation were shown in Figure 12 and Table 5. From the injection of tumor cells to the 24th day, the order of tumor growth speed and size was: Lys+adj group>Lys+Th+Adj group. The size of the tumors on the 17th day was shown in Figure 12, and the mean tumor volume (mean+SEM) was: 419.9±165 mm³ and 154.9±111.6 mm³, respectively.

**Table 5**

| Group | Administered Substance | Tumor volume (mean+SEM, mm³) | Relative tumor volume (%) |
|---|---|---|---|
| Lys+Adj | B16F10 Lys+adj | 419.9±165 | 100% |
| Lys+Th+adj | B16F10 Lys+Th+adj | 154.9±111.6 | 36.9% |

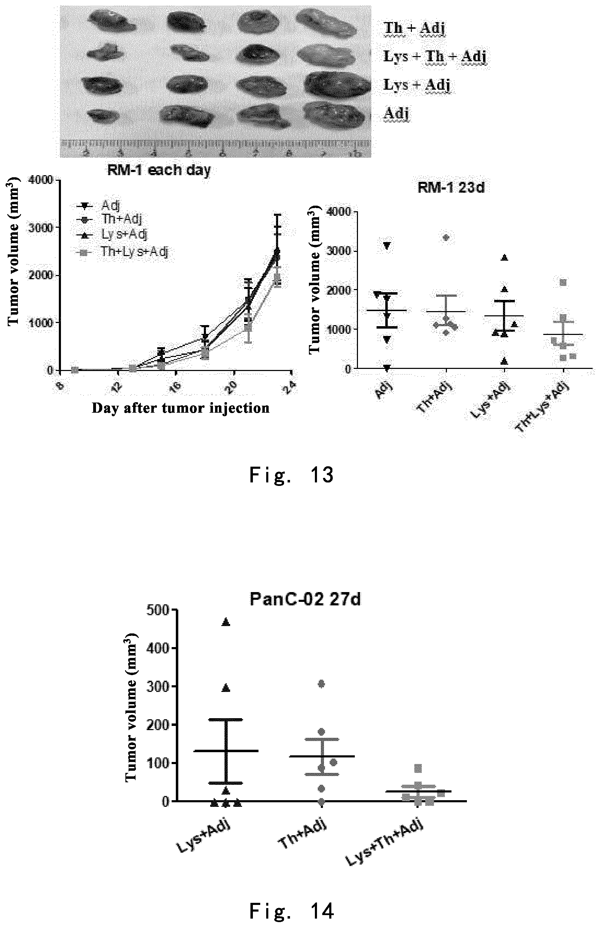

The results of prostate cancer RM-1 cell inoculation were shown in Figure 13 and Table 6. From the injection of tumor cells to the 24th day, the order of tumor growth speed and size was: Adj group>Th+Adj group>Lys+Adj group>Lys+Th+Adj group. The size of the tumors on the 24th day was shown in Figure 13, and the mean tumor volume (mean±SEM) was: 1476±436.9 mm³, 1457±377.1 mm³, 1335±384.6 mm³, and 881.3±301.3 mm³, respectively.

**Table 6**

| Group | Administered Substance | Tumor volume (mean+SEM, mm³) | Relative tumor volume (%) |
|---|---|---|---|
| Adj | adj | 1476±436.9 | 100% |
| Th+Adj | Th+adj | 1457±377.1 | 98.7% |
| Lys+Adj | RM-1 Lys+Adj | 1335±384.6 | 90.4% |
| Lys+Th+Adj | RM-1 Lys+Th+adj | 881.3±30 1.3 | 59.7% |

The results of pancreatic cancer PanC-02 cells were shown in Figure 14 and Table 7. On the 27th day after injection of tumor cells, the order of tumor size was: Lys+adj group>Th+adj group>Lys+Th+Adj group. The mean tumor volume (mean±SEM) was 0.4475±0.04211 mm³ and 0.235±0.02958 mm³, respectively.

**Table 7**

| Group | Administered Substance | Tumor volume (mean+SEM, mm³) | Relative tumor volume (%) |
|---|---|---|---|
| Lys+Adj | PanC-02+adj | 133.5±82.74 | 100% |
| Th+Adj | Th+adj | 119.3±45.45 | 89.4% |
| Lys+Th+Adj | PanC-02 Lys+Th+adj | 27.72±13.62 | 20.8% |

The results showed that the immune enhancer UThE had inhibitory effect on a variety of tumors. According to the current tumor types in the laboratory, we can conclude that UThE may have inhibitory effects on various tumors, that is, the tumor inhibition of UThE is broad-spectrum.

### Example 7 The relationship between the dose of UThE and its tumor inhibitory effect

The method of this example was basically the same as those of Example 1, Example 2, and Example 6. The difference was that the experimental protocol was a B16F10 tumor treatment model, and only low to high doses of UThE combined with adjuvant Adj were used to treat tumors in mice. The vaccine was injected subcutaneously at 2 sites at the root of the hind limbs of each mouse, with 50 microliters per site. The four immunization doses were the same, each 50 microliters of vaccine components were adjuvant (including 25 microliters of MF59, 25 µg CpG, and 12.5 µg PolyI:C) and UThE, and the doses of UThE were 30 µg, 50 µg and 80 µg, respectively.

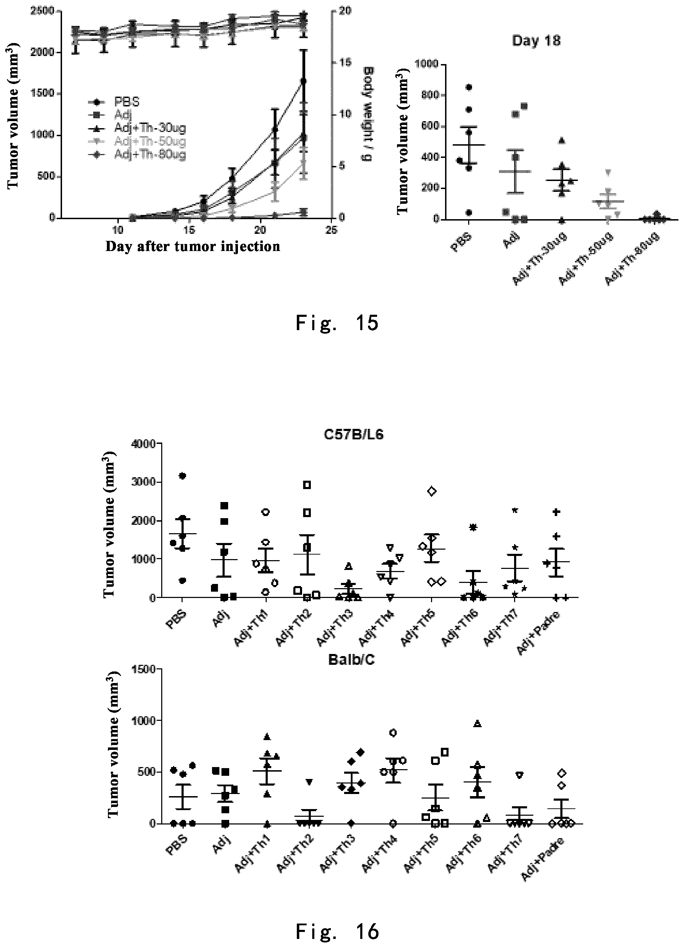

The tumor results of each group after treatment were shown in Figure 15 and Table 8. From the injection of tumor cells to the 23rd day, the body weight of mice in each group was normal and showed a steady increase. The order of tumor growth speed and size was: PBS group>Adj group>Adj+Th-30µg group>Adj+Th-50µg group>Adj+Th-80µg group. The mean tumor volume (mean±SEM) on day 18 was: 480.4±118.6 mm³, 309.9±139.6 mm³, 253.3±70.59 mm³, 115.8±45.05 mm³, and 5.753±5.743 mm³, respectively.

**Table 8**

| Group | Substance to be administered | Tumor volume (18d) (mean+SEM, mm³) | Relative tumor volume (%) |
|---|---|---|---|
| PBS | PBS | 480.4±118.6 | 100% |
| Adj | adj | 309.9±139.6 | 64.5% |
| Adj-Th30 | Adj+Th 30µg | 253.3±70.59 | 52.7% |
| Adj-Th50 | Adj+Th 50µg | 115.8±45.05 | 24.1% |
| Adj-Th80 | Adj+Th 80µg | 5.743±5.743 | 1.20% |

The results showed that UThE combined with appropriate adjuvant could achieve tumor inhibitory effect, and this inhibitory effect was enhanced with the increase of UThE dosage until the content of UThE increased to 80 µg, the weight of the mice was not affected, and no toxicity was observed. The tumor inhibition rate reached 98.8% at 80 µg. It is suggested that after UThE enters the body, it can take the initiative to acquire the tumor antigens existing in the body, and generate antigen-specific CTLs to achieve tumor killing and tumor inhibition.

### Example 8 Inhibitory effect of UThE on tumors in different strains of mice

The administration method was the same as in Example 7. However, unlike Example 7, in which the dose-effect relationship of UThE was investigated, the purpose of Example 8 was to investigate the inhibitory effect of each polypeptide on tumors in different stains of mice at the same dose of 50 µg. The dose of each peptide of DThE1 to DThE7 was 50 µg, and the dose of adjuvant was the same as that of Example 7. C57BL/6 mice were inoculated with 10⁵ B16F10 cells, and Balb/C mice were inoculated with 10⁴ 4T1 cells. On the third day after tumor inoculation, the mice were immunized, and the tumor growth of the mice in each group was shown in Figure 16. The tumor inhibition effect of each polypeptide on the two strains of mice was different, which may be related to the MHC II type of the two strains of mice, suggesting that MHC-related typing might be considered in the later stage of administration to carry out targeted immunization.

All documents mentioned in the present invention are incorporated by reference herein as if each document were incorporated separately by reference. Furthermore, it should be understood that after reading the foregoing teachings of the invention, various changes or modifications may be made to the invention by those skilled in the art and that these equivalents also fall in the scope of the claims appended to this application.

## Claims

1. A tumor immune enhancer, which comprises one or more polypeptides having the structure of formula I, or a pharmaceutically acceptable salt thereof:
Z0-Z1-Z2 (I)
wherein,
Z0 is none, or a peptide segment consisting of 1-10 amino acid residues;
Z2 is none, or a peptide segment consisting of 1-10 amino acid residues;
Z1 is a peptide segment selected from the group consisting of:
(a) a polypeptide as shown in SEQ ID NOs: 1-9;
(b) a derivative polypeptide which is formed by substitution, deletion or addition of one, two or three amino acid residues in amino acid sequences of SEQ ID NOs: 1-9, and which can bind to human and murine type II histocompatibility antigens.

2. The tumor immune enhancer of claim 1, wherein Z0 and Z2 contain at least 2-8, preferably 3-6 hydrophilic amino acid residues.

3. The tumor immune enhancer of claim 1, wherein at least one of Z0 and Z2 contains (Arg)n structure, and wherein n is a positive integer of 3-6.

4. The tumor immune enhancer of claim 1, wherein the tumor immune enhancer has an activity of enhancing tumor immunity.

5. A multimer, which is composed of m monomers in series connection, and has a function of enhancing tumor immunity, wherein, m is a positive integer ≥ 2, and each monomer independently has the structure of formula I:
Z0-Z1-Z2 (I)
wherein,
Z0 is none, or a peptide segment consisting of 1-10 amino acid residues;
Z2 is none, or a peptide segment consisting of 1-10 amino acid residues;
Z1 is a peptide segment selected from the group consisting of:
(a) a polypeptide as shown in SEQ ID NOs: 1-9;
(b) a derivative polypeptide which formed by substitution, deletion or addition of one, two or three amino acid residues in amino acid sequences of SEQ ID NOs: 1-9, and which can bind to histocompatibility antigens.

6. An isolated nucleic acid molecule, which encodes a polypeptide, wherein the polypeptide has the structure of formula I, or the polypeptide is a multimer composed of m monomers in series connection, wherein each monomer independently has the structure of formula I, and m is a positive integer >2.

7. A pharmaceutical composition comprising:
(a) a tumor immune enhancer or a pharmaceutically acceptable salt thereof of claim 1, or a multimer of claim 5, and/or an isolated nucleic acid molecule of claim 6; and
(b) a pharmaceutically acceptable carrier or excipient.

8. The pharmaceutical composition of claim 7, wherein the pharmaceutical composition further comprises:
(c) a tumor antigen.

9. The pharmaceutical composition of claim 8, wherein the pharmaceutical composition further comprises:
(d) a DC activator.

10. The pharmaceutical composition of claim 7 or 8, wherein the pharmaceutical composition is a tumor vaccine composition.

11. A use of a substance, which selected from the group consisting of: a tumor immune enhancer or a pharmaceutically acceptable salt thereof of claim 1, or a multimer of claim 5, or an isolated nucleic acid molecule of claim 6, wherein the substance is used for preparing a drug for improving an antitumor activity of a tumor antigen, or for preparing an antitumor vaccine composition.

12. A kit, comprising:
(i) a first container, and a tumor immune enhancer of claim 1, or a drug containing the tumor immune enhancer located in the first container;
(ii) a second container, and a tumor-treating drug located in the second container; and
(iii) an specification, in which an instruction for the combined administration of the tumor immune enhancer and the tumor-treating drug for cancer treatment is described.

13. A method of treating cancer, comprising the steps of:
(i) administering a tumor immune enhancer or a pharmaceutically acceptable salt thereof of claim 1, or a multimer of claim 5, or an isolated nucleic acid molecule of claim 6, or a pharmaceutical composition of claim 7 to a subject in need; and
(ii) administering a tumor-treating drug to the subject in need.
